**Europäisches Patentamt**

(19) **European Patent Office**

**Office européen des brevets**

(11) Publication number : **0 314 761 B1**

# EUROPEAN PATENT SPECIFICATION

(45) Date of publication of patent specification :
**17.06.92 Bulletin 92/25**

(51) Int. Cl.⁵ : **A61L 9/03, A61L 9/04**

(21) Application number : **88904879.9**

(22) Date of filing : **12.05.88**

(86) International application number :
**PCT/US88/01570**

(87) International publication number :
**WO 88/08721 17.11.88 Gazette 88/25**

(54) **VAPOR CIRCULATION APPARATUS.**

(30) Priority : **14.05.87 US 50162**

(43) Date of publication of application :
**10.05.89 Bulletin 89/19**

(45) Publication of the grant of the patent :
**17.06.92 Bulletin 92/25**

(84) Designated Contracting States :
**AT BE CH DE FR GB IT LI LU NL**

(56) References cited :
**No further relevant documents have been disclosed.**

(73) Proprietor : **SANI-FRESH INTERNATIONAL, INC.**
**4702 Goldfield Drive**
**San Antonio Texas 78218 (US)**

(72) Inventor : **NORMAN, Richard, O.**
**805 Ridgemont**
**San Antonio, TX 78209 (US)**

(74) Representative : **McCall, John Douglas et al**
**W.P. THOMPSON & CO. Coopers Building**
**Church Street**
**Liverpool L1 3AB (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a vapor circulation apparatus for holding a predetermined volume of liquid material, such as an aromatic oil, for evaporation and for circulating the evaporated matter or fragrance throughout a room. More particularly, this invention relates to a vapor circulation apparatus wherein the liquid to be evaporated is held in a sealed container and wherein the vapor circulation apparatus automatically and periodically unseals the container, withdraws a predetermined volume of the liquid to be evaporated, and then reseals the container.

U. S. Patent 3, 990, 848-Corris discloses a vapor circulation device for distributing a deodorizer or an insecticide in vaporous form. The product to be vaporized is a gel that is packaged in a porous container which is placed in the vapor circulation device. The vapor circulation device also includes a fan which induces air flow past the gel thereby causing sublimed vapors to be circulated out of the housing into the environment.

U. S. Patent 3, 804, 592-Garbe discloses a vapor circulation device wherein a deodorant is provided in liquid form and is dispersed as a droplet onto a blotter from which the liquid is allowed to evaporate. A fan circulates the evaporated material throughout the environment. Garbe, at column 2, lines 5-10, discloses that by using a variety of inserts with the dispensing opening of the container, one can selectively choose the rate of droplet flow from the container. It is believed that the evaporation time for a given size droplet would be increased if the droplet were not allowed to be dispersed in a blotter. Thus, by eliminating the blotter, a given volume of the liquid being vaporized would last longer.

One disadvantage of the gel deodorizer of Corris is that the gel is continuously exposed to the atmosphere. Since the deodorizer is made up of several materials having different evaporation rates and because the fragrance bearing materials generally evaporate faster than the other materials, the perceived fragrance diminishes over time. Even though the liquid drop deodorizer of Garbe employs a wick element to control the drop rate, because the container is not completely sealed, some of the fragrance bearing materials will evaporate from the reservoir supply.

In U. S. Patents 3,617, 214-Dolac and 3, 739, 944-Rogerson, the material that is introduced as a vapor into the environment is packaged in liquid form in a pressurized aerosol container. The spray nozzle of the aerosol container is periodically operated which sprays the product into the environment. One disadvantage of using an aerosol spray is that the atomized liquid particles fall to the floor where they can be stepped on thereby losing their effectiveness as a vapor emitting material.

U.S. Patent 3,085,719-Weber discloses a deodorizer that utilizes a periodically activated drop dispenser. As described at column 5, lines 7-14, the drops are deposited on a heated element where the liquid is heated above its boiling point thereby introducing the vapors into the environment. To maintain the fragrance level in a room, Weber at column 5, lines 32 -34 describes drop rates of up to 10 drops per minute.

It is an object of this invention to provide a vapor circulation apparatus, such as an air freshener, wherein the air treatment product is an evaporated liquid.

It is another object of this invention to provide a vapor circulation apparatus wherein a liquid to be evaporated is held in a sealed container and wherein a predetermined volume of the liquid to be evaporated is withdrawn from the container and the container is then resealed.

And yet another object of this invention is to package a liquid air freshener product in a disposable refill cartridge.

In accordance with this invention, there is provided a vapor circulation apparatus comprising;

a main housing having a first air opening therein and a refill unit housing having a second air opening therein, said refill unit housing being connected to said main housing;

a sealed container comprising a liquid reservoir, mounted in the refill unit housing;

a dosing valve housing which extends from the liquid reservoir;

a dosing pin including a dosing element on one end thereof for collecting a fixed volume of the liquid for evaporation, the dosing pin residing within said dosing valve housing such that the dosing element normally extends beyond the dosing valve housing;

actuating means for periodically inserting the dosing element into the dosing valve housing and withdrawing the dosing element therefrom with said fixed volume of liquid;

means for resealing the container after the dosing element has been withdrawn; and

means in the main housing for periodically inducing air flow through said air openings after the container has been resealed.

In another aspect of the invention, the container comprises a liquid reservoir, a dosing element housing connected to the reservoir for receiving the dosing element, and a membrane seal, located between the reservoir and the dosing element housing, for providing a sealed reservoir.

In yet another aspect of the invention, dosing element includes a piercing member adjacent the membrane for puncturing the membrane to allow liquid to flow into the dosing element housing.

According to the present invention, there is also provided a disposable cartridge contained within a refill unit housing having a first air opening therein, said cartridge being adapted for use in a vapor circulation apparatus, said disposable cartridge. comprising:-

a sealed container comprising a liquid reservoir;

a dosing valve housing extending from the liquid reservoir

a dosing pin including a dosing element on one end thereof for collecting and withdrawing a fixed volume of liquid from said sealed container for evaporation, the dosing pin residing within said dosing valve housing such that the dosing element normally extends beyond the dosing valve housing, the actuating means periodically reciprocating said dosing pin within said valve housing such that said dosing element is inserted into said dosing valve housing and withdrawn therefrom with said fixed volume of liquid;

means for resealing the container after the dosing element has been withdrawn.

The refill cartridge can have either the air intake or air outlet opening of the completed vapor circulation device. Batteries to power the fan and the means for inserting the dosing pin into the container can also be packaged in the disposable refill cartridge.

While the specification concludes with claims particularly pointing out and distinctly claiming that which is regarded as the present invention, the objects and advantages of this invention can be more readily ascertained from the following description of a preferred embodiment when read in conjunction with the accompanying drawings in which:

Fig. 1 is a perspective view of one embodiment of the vapor circulation apparatus showing a main housing detached from a disposable refill unit;

Fig. 2 is a front cross-sectional view of the vapor circulation apparatus with an attached refill unit in the operating position;

Fig. 3 is an exploded perspective view of the disposable refill unit;

Fig. 4 is a perspective view of the dosing pin for withdrawing a predetermined volume of liquid from the sealed container in the refill unit; and

Fig. 5 is a schematic diagram of the electronic circuits which control the vapor circulation apparatus.

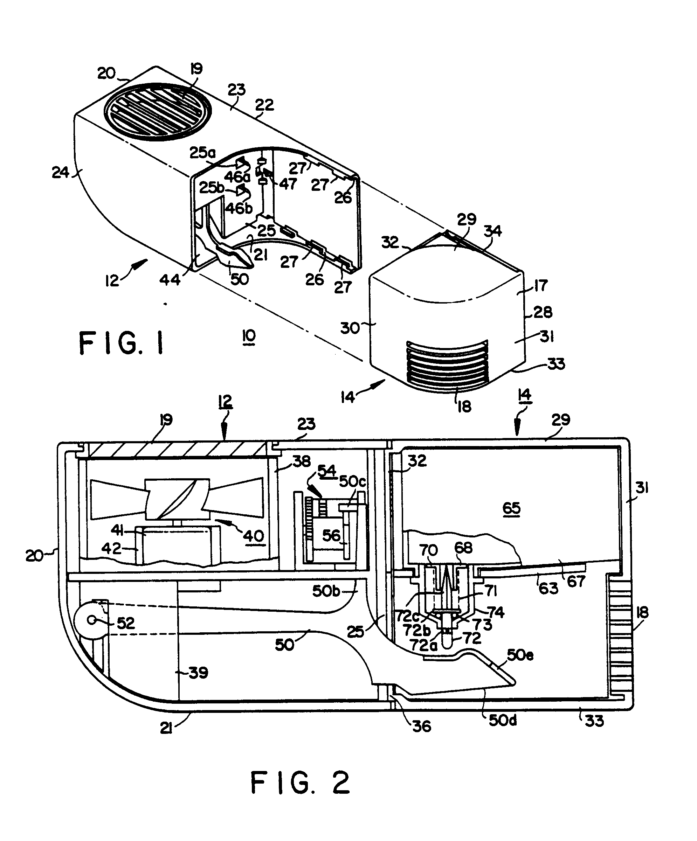

For the sake of convenience, elements described with reference to a specific figure will retain the same reference designation in the description of subsequent figures. Referring now to Fig. 1, a vapor circulation apparatus 10 is shown, which in one preferred embodiment can be disassembled into a main dispenser unit 12 and a disposable refill unit 14. The housing of the main dispenser unit 12 comprises sidewall 20, bottom wall 21, rear wall 22, top wall 23, and front wall 24. Mounted within the housing of the main dispenser unit is an interior sidewall 25. The housing of the disposable refill unit 14 comprises rear wall 28, top wall 29, front wall 30, sidewall 31, inner sidewall 32 and bottom wall 33.

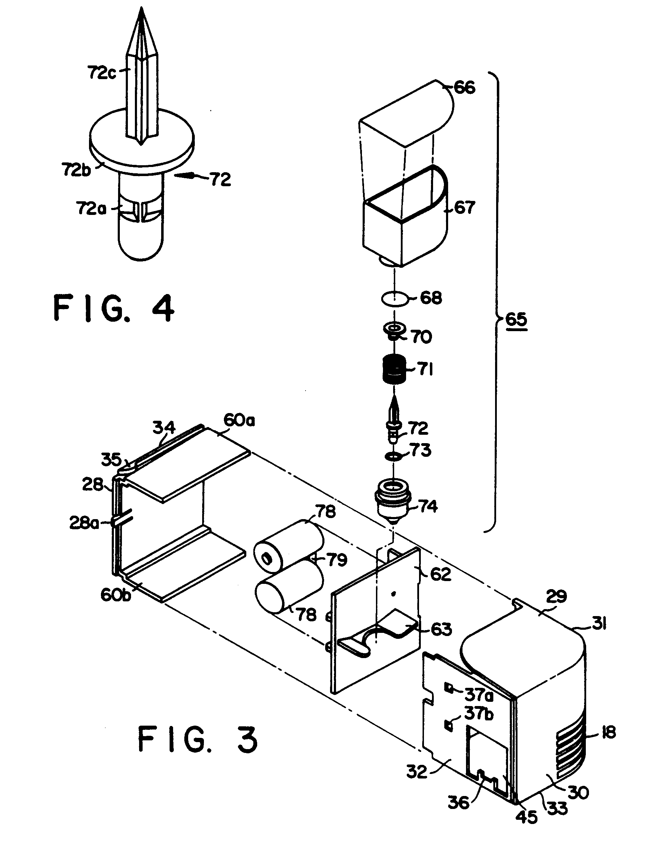

Three steps 27 on the bottom wall 21 of the main dispenser unit 12 are spaced from the extended rear wall 22 of the main dispenser unit 12 to form a channel 26 at the lower corner of the extended rear wall 22. Three similar steps 27 extending downward from the top wall 23 of the main dispenser unit 12 form a similar channel 26 at the upper corner of the extended rear wall 22. As best shown in Fig. 3, a rail 34 is formed at the upper rear wall 28 corner and lower rear wall 28 corner (not shown) of the disposable refill unit 14. The end of each rail 34 can be pointed and gradually increase to have a thickness greater than the rail 34 but less than the width of channel 26 to form a latch 35 at the end thereof. To assemble the disposable refill unit 14 to the main dispenser unit 12, upper and lower rails 34 are inserted into channels 26 and as the rails 34 slide in their respective grooves 26, the alignment of the disposable refill unit 14 is maintained as it is assembled with the main dispenser unit 12. When refill unit 14 is assembled into the main dispenser 12, ramp 47, shown in Fig. 1, exerts a force to the back of spring finger 28a on rear wall 28 of the refill unit 14 which locates latch 35 beyond the width of channel 26 and the application of a lateral force only to disassemble refill unit 14 will cause latches 35 to engage the innermost steps 27 of the main dispenser unit 12. To disassemble the refill unit 14 from the main dispenser unit 12, pressure is applied to front wall 30 of refill unit 14 which locates latch 35 entirely within channel 26 while applying a lateral force to refill unit 14 thereby causing rails 34 to slide in channels 26 to remove the refill unit 14. If desired the assembled vapor circulation apparatus 10 can be mounted to a wall or other surface using well known means such as double sided adhesive or screws.

Referring now to Fig. 2 which shows the disposable refill unit 14 assembled with the main dispenser unit 12, the composite vapor circulation apparatus housing has an air intake opening 18 at the lower corner of the disposable refill unit 14 and an air outlet opening 19 located in the top wall 23 of the main dispenser unit 12.

The air outlet opening 19 is in the form of a louvered disk which is rotatably mounted in the top wall 23 so that the direction of air flow from the vapor circulation apparatus 10 can be controlled. If it is necessary for the vapor circulation apparatus 10 to have interior vertical walls such as interior sidewall 25 of the main dispenser unit 12 and interior sidewall 32 of the disposable refill unit 14, the interior sidewall 25 is constructed to have an opening 44 (best shown in Fig. 1) which corresponds with an opening 45 (best shown in Fig. 3) in interior sidewall 32 of the disposable refill unit 14 in order to provide an air flow path between the air intake opening 18 and air outlet opening 19.

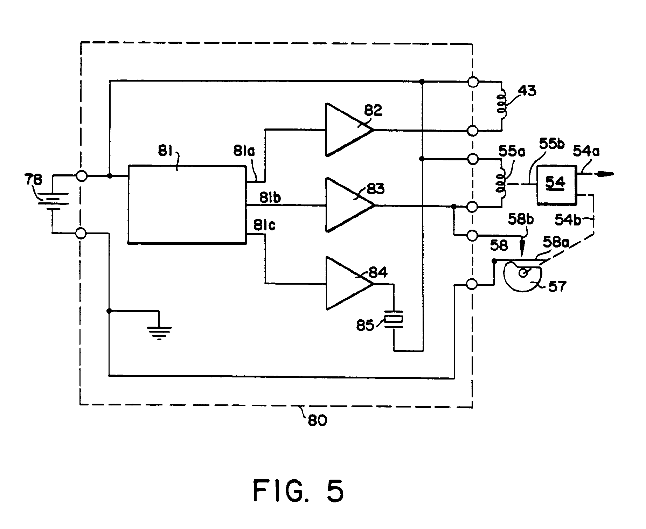

As shown in Fig. 2, the main dispenser unit 12 also includes a fan 40 for pulling air into the air intake opening 18 and expelling air out of air outlet opening 19; an actuator 50 that is pivotally mounted at one end about pivot 52 for causing a drop of liquid to be withdrawn from a container 65 in the disposable refill unit 14; means 54 for periodically operating the actuator 50; and electronics on a printed circuit board 80, shown schematically in Fig. 5, for controlling the operation of the fan 40 and the means 54 for operating the actuator 50. The fan 40 is mounted within a fan shroud 38 and the fan motor 41 is mounted in motor housing 42. The pivotally mounted actuator 50 includes a vertical leg member 50b which terminates in a shoulder 50c. The means 54 for periodically operating the actuator 50 includes an actuator motor 55 (shown schematically in Fig. 5) which drives a gear train and attached cam 56 which contacts the lower surface of shoulder 50c to impart vertical motion to the shoulder 50c. Gam 56 makes one revolution for 87.5 revolutions of the shaft output of actuator motor 55.

The refill unit includes a container 65 of liquid to be evaporated and batteries 78 (best shown in Fig. 3) for driving the electronics on printed circuit board 80, the fan 40 and the means 54 for operating actuator 50.

Referring now to Fig. 3, the rear wall 28 of the disposable refill unit 14 includes upper extension 60a which fits under the top wall 29 and lower extension 60b which fits over the bottom wall 33 of the refill housing. The disposable refill unit 14 also has a vertical support wall 62 for supporting two batteries 78 which are connected in series by means of shorting bar 79. Also mounted to the vertical support wall 62 is a shelf 63 for holding the container 65 of liquid to be evaporated. Referring now to Figs. 2 and 3, the sealed container 65 comprises a liquid reservoir 67, the top of which is sealed with a metal foil 66. Also forming a part of the liquid container 65 is a dosing valve housing 74 which contains therein an O-ring seal 73, a dosing pin 72, a compression spring 71 and an upper spring retaining disk 70. As best shown in Fig. 4, the lower portion of dosing pin 72 has a diameter of 0.381 centimeters (0.150 inches) and has four wedges each capable of holding about 0.016 cubic centimeters (0.001 cubic inches) of liquid to be evaporated. The central portion of dosing pin 72 is disc-shaped to provide a lower support 72b for compression spring 71. The upper portion of dosing pin 72 forms a piercing member 72c. Referring again to Figs. 2 and 3, the upper compression disk 70 is pressed into the dosing valve housing 74 over a plurality of bosses (not shown) near the upper edge of the dosing pin housing 74 and the upper opening of dosing pin housing 74 is sealed with foil membrane 68. The upper spring retaining disk 70 has an elongated axial opening which fits over the piercing member 72c to guide the piercing of metal foil seal 68. The sealed dosing pin housing assembly is then press fit into an opening in the lower wall of the liquid reservoir 67 to provide a completely sealed liquid container. When the refill support wall 62 with the liquid container 65 and battery 78 mounted thereon is assembled into the refill unit 14, the battery electrodes will line up with holes 37a and 37b in inner sidewall 32 of the refill unit 14 and dosing pin 72 will be located in the air flow path between air intake opening 18 and opening 45 in interior sidewall 32.

Referring now to Fig. 5, the control electronics for the fan 40 and the actuator 50 are contained on printed circuit board 80 mounted within the main dispenser unit 12. The control is provided by a four-bit microprocessor integrated circuit 81 that has been specially designed by means of the masks used to fabricate the integrated circuit 81 to provide the desired control outputs. Control output 81a of the microprocessor 81 is applied to an amplifier 82 which drives the coil 43 of fan motor 41. Control output 81a is a repetitive signal that turns the fan on for 75 seconds and turns the fan off for 75 seconds. Control output 81b of microprocessor 81 is applied to amplifier 83 which drives the coil 55a of actuator motor 55. Control signal 81b is a signal that operates the actuator motor coil 55a for a half second every 20 minutes with the additional requirement that when battery 78 is initially connected to the printed circuit board 80, the actuator motor 55 is operated for 0.5 seconds every 1.5 seconds for 8 cycles. Since the liquid container 65 holds a two-month supply of liquid to be evaporated, microprocessor 81 is designed to operate the fan motor 41 and the actuator motor 55 for about 56 days after which the fan motor 41 and the actuator motor 55 are disabled.

The actuator drive means 54 has a second shaft output 54b which drives cam 57 and controls the opening and closing of switch 58. The function of cam 57 and switch 58 is to make sure that the actuator drives mean 54 stops at approximately the same point in a cycle. The actuator motor control signal from amplifier 83 operates actuator motor 55 for a time that is less than the time that it takes for the actuator drive 54 to complete one full dosing cycle which can vary from about 0.75 to 1.0 seconds depending on the battery 78 voltage. The shape of cam 57 is such that shortly after the actuator motor 55 is energized, cam 57 will close switch contacts 58a and 58b thereby connecting ground to the end of the actuator motor 55a that is actually driven by amplifier 83 so that when amplifier 83 turns off before the completion of a dosing cycle, the ground connection through switch 58 keeps the coil 55a of actuator motor 55 energized. The actuator motor 55 will continue to operate until the shape of cam 57 allows switch contacts 58a and 58b to open thereby removing power from the actuator motor 55. Thus, the beginning and end of a dosing cycle is really controlled by the shape of cam 57 and not by the length of time that amplifier 83 is turned on.

Control output 81c of microprocessor 81 is applied to amplifier 84 which drives piezo alarm element 85. The piezo alarm element 85 is driven by a 4096 cycle square have burst lasting 0.0625 seconds. When a refill cartridge 14 is initially assembled with the main dispenser unit 12, eight such 4096 cycle square wave bursts are spaced equally over one second, then the piezo alarm element 85 is turned off for three seconds, and then another eight bursts are generated. After the microprocessor 81 has timed the 56 days of operation and has disabled the fan motor 41 and the actuator motor 55, the piezo alarm element 85 is then activated from time to time over a four-day period to signal that the disposable refill unit should be replaced. At the end of those four days, the microprocessor 81 will not again activate the piezo alarm element 85 unless the disposable refill unit 14 is replaced.

What now follows is a brief description of the use and operation of the vapor circulation apparatus 10 through a complete life cycle of a disposable refill unit 14. To assemble the refill unit 14 with the main dispenser unit 12, rails 34 of the refill unit housing are placed into the channels 26 formed at the extended portion of rear wall 22 of the main dispenser unit 12. Referring to Fig. 2, as the refill unit 14 is pushed into assembly with the main dispenser unit 12, the sloped surface 50e of actuator 50 contacts the bottom of dosing pin 72. As the refill unit 14 is further pushed into assembly with the main dispenser unit 12, the combination of the sloped surface 50e of actuator 50 and the action of step 36 formed in inner sidewall 32 of the refill unit 14 on the lower inclined surface 50d of actuator 50 exerts an upward force on the dosing pin 72 allowing the upper piercing member 72c of the dosing pin 72 to break foil membrane 68 which will allow liquid in the reservoir 67 portion of container 65 to flow into dosing pin housing 74. When the assembly of the refill unit 14 with the main dispenser unit 12 is completed, electrical contacts 46a and 46b (shown in Fig. 1) will extend through holes 25a and 25b in sidewall 25 of main unit 12 and through corresponding holes 37a and 37b in the inner sidewall 32 of the refill unit 14, as shown in Fig. 3, and make contact with the electrodes of battery 78 thereby providing power to printed circuit board 80 which initiates a 60-day timing cycle for controlling the fan motor 41, the actuator motor 55 and the piezo alarm element 85. Approximately two seconds after the microprocessor 81 has been first activated, the piezo alarm element 85 is operated for eight seconds as previously described so that the person assembling the refill unit 14 to the main dispenser unit 12 can be provided with some indication that the printed circuit board 80 is functioning properly. Also, two seconds after the microprocessor 81 has been energized, the actuator motor 55 is energized eight times during the next eleven seconds thereby causing dosing pin 72 and its piercing member 72c to be respectively inserted into the dosing pin housing 74 and fluid reservoir 67 and then withdrawn eight times which primes the dosing valve housing 74 so that it becomes filled with the liquid to be evaporated. After the eighth of these priming cycles, the four wedges 72a of dosing pin 72 will each contain about 0.016 cubic centimeters (0.001 cubic inch) of liquid for evaporation and subsequent circulation by the vapor distribution apparatus 10. Due to the action of compression spring 71 which returns dosing gin 72 to the withdrawn position and maintains pressure on O-ring seal 73, the container 65 is effectively resealed after the dosing pin 72 has been withdrawn from the dosing pin housing 74. Because of this resealing, the various components of the liquid cannot evaporate from the container 65 and the effectiveness of the liquid is maintained over the two-month expected life of the refill unit 14. Seventy-seven seconds after the refill unit 14 is assembled to the main dispenser unit 12, the microprocessor 81 turns on the fan for 75 seconds and continues the 75 second on, 75 second off periodic operation of the fan 40 for 56 days. Twenty minutes after the refill unit 14 is assembled to the main dispenser unit 12, the microprocessor 81 will insert dosing pin 72 into dosing pin housing 74, withdraw another sample of liquid to be evaporated, and reseal the container 65. This sampling rate has been determined by the evaporation rate of the liquid which is such that about 90% of the sample evaporates in 20 minutes. This 20-minute sampling period will continue for 56 days. At the end of the 56-day period the actuator motor 55 and the fan motor 41 will be disabled, that is, they will not be energized any longer. The microprocessor 81 will then periodically activate the piezo alarm element 85 as a signal that the refill unit 14 needs replacement. At the end of the 60th day from the assembly of the refill unit 14 with the main dispenser unit 12, the microprocessor 81 will also disable the piezo alarm element 85.

While the present invention has been described with respect to a specific embodiment thereof, it will be obvious that various changes and modifications may be made without departing from the invention in its broader aspects. For example, both the air intake opening and the air outlet opening can be formed in the housing of the main dispenser unit.

## Claims

1. A vapor circulation apparatus comprising;

a main housing (12) having a first air opening (19) therein and a refill unit housing (14) having a second air opening (18) therein, said refill unit housing (14) being connected to said main housing (12);

a sealed container comprising a liquid reservoir (65), mounted in the refill unit housing (14);

a dosing valve housing (74) which extends from the liquid reservoir (65);

a dosing pin (72) including a dosing element (72a) on one end thereof for collecting a fixed volume of the liquid for evaporation, the dosing pin (72) residing within said dosing valve housing (74) such that the dosing element (72a) normally extends beyond the dosing valve housing (74);

actuating means (50) for periodically inserting the dosing element (72a) into the dosing valve housing (74) and withdrawing the dosing element (72a) therefrom with said fixed volume of liquid;

means (78) for resealing the container (65) after the dosing element (72a) has been withdrawn; and

means (49) in the main housing (12) for periodically inducing air flow through said air openings (18,19) after the container has been resealed.

2. A vapor circulation apparatus according to claim 1 wherein the means for periodically inducing air flow is a periodically activated fan (49).

3. A vapor circulation apparatus according to claim 1 or claim 2 wherein the sealed container comprises a frangible seal (68) located between the reservoir (67) and the dosing valve housing (74) for providing a sealed reservoir, said dosing pin (72) including a piercing member (72c), said piercing member (72c) puncturing said frangible seal (68) on the initial insertion of said dosing element (72a) into said dosing valve housing (74) allowing liquid to flow from said liquid reservoir (67) into dosing valve housing (74).

4. A vapor circulation apparatus according to any preceding claim wherein liquid container (65) and dosing pin (72) are packaged in a disposable refill cartridge.

5. A vapor circulation apparatus according to claim 4 wherein said refill unit housing (14) encloses and is part of said disposable refill cartridge.

6. A disposable cartridge contained within a refill unit housing (14) having a first air opening (18) therein, said cartridge being adapted for use in a vapor circulation apparatus according to any preceding claim, said disposable cartridge comprising:-

a sealed container comprising a liquid reservoir (65);

a dosing valve housing (74) extending from the liquid reservoir (65)

a dosing pin (72) including a dosing element (72a) on one end thereof for collecting and withdrawing a fixed volume of liquid from said sealed container (65) for evaporation, the dosing pin (72) residing within said dosing valve housing (74) such that the dosing element (72a) normally extends beyond the dosing valve housing (74), the actuating means (50) periodically reciprocating said dosing pin (72) within said valve housing (74) such that said dosing element (72a) is inserted into said dosing valve housing (74) and withdrawn therefrom with said fixed volume of liquid;

means (73) for resealing the container (65) after the dosing element (72a) has been withdrawn.

7. A disposable cartridge according to claim 6 wherein said sealed container comprises a frangible seal (68) located between the reservoir and the dosing valve housing for providing a sealed reservoir, (65) said dosing pin (72) further including a piercing member (72c), said piercing member (72c) puncturing said frangible seal (68) on the initial insertion of said dosing element (72a) into said dosing valve housing (74) thereby allowing liquid to flow from said liquid reservoir via gravity into said dosing valve housing (74).

8. A disposable cartridge according to claim 6 or claim 7 further comprising means (34) for aligning the cartridge as it is attached to the main housing (12) of the vapor circulation apparatus.

**Patentansprüche**

1. Dampfzirkulationsvorrichtung enthaltend:

ein Hauptgehäuse (12) mit einer darin angeordneten ersten Luftöffnung (19) und ein Nachfülleinheitsgehäuse (14) mit einer darin angeordneten zweiten Luftöffnung (18), wobei das Nachfülleinheitsgehäuse (14) mit dem Hauptgehäuse (12) verbunden ist;

einen ein Flüssigkeitsreservoir (65) enthaltenden abgedichteten Behälter, der in dem Nachfülleinheitsgehäuse (14) angeordnet ist;

ein Dosierventilgehäuse (74), das sich von dem Flüssigkeitsreservoir (65) erstreckt;

einen Dosierzapfen (72) mit einem Dosierelement (72a) an seinem einen Ende zum Sammeln eines festen Volumens der Flüssigkeit zur Verdampfung, wobei der Dosierzapfen (72) in dem Dosierventilgehäuse (74) derart angeordnet ist, daß sich das Dosierelement (72a) normalerweise über das Dosierventilgehäuse (74) hinaus erstreckt;

Betätigungsmittel (50) zum periodischen Einschieben des Dosierelements (72a) in das Dosierventilgehäuse (74) und zum Zurückziehen des Dosierelementes (72a) aus diesem mit dem festen Flüssigkeitsvolumen;

Mittel (78) zum Wiederabdichten des Behälters (65) nach dem Zurückziehen des Dosierelementes (72a); und

Mittel (49) in dem Hauptgehäuse (12) zum periodischen Erzeugen von Luftströmung durch die Luftöffnungen (18, 19) nach dem Wiederabdichten des Behälters.

2. Dampfzirkulationsvorrichtung nach Anspruch 1, bei der die Mittel zum periodischen Erzeugen der Luftströmung ein periodisch betätigter Lüfter (49) sind.

3. Dampfzirkulationsvorrichtung nach Anspruch 1 oder 2, bei der der abgedichtete Behälter eine zwischen dem Reservoir (67) und dem Dosierventilgehäuse (74) angeordnete durchstoßbare Dichtung (68) zur Schaffung eines abgedichteten Reservoirs aufweist und der Dosierzapfen (72) ein Durchstoßelement (72c) enthält, das die durchstoßbare Dichtung (68) beim erstmaligen Einschieben des Dosierelements (72a) in das Dosierventilgehäuse (74) punktiert, was es ermöglicht, daß Flüssigkeit von dem Flüssigkeitsreservoir (67) in das Dosierventilgehäuse (74) fließt.

4. Dampfzirkulationsvorrichtung nach einem vorhergehenden Anspruch, bei der der Flüssigkeitsbehälter (65) und der Dosierzapfen (72) in einer Wegwerf-Nachfüllkartusche verpackt sind.

5. Dampfzirkulationsvorrichtung nach Anspruch 4, bei der das Nachfülleinheitsgehäuse (14) die wegwerfbare Nachfüllkartusche einschließt und einen Teil von ihr bildet.

6. Wegwerfkartusche in einem Nachfülleinheitsgehäuse (14) mit einer ersten darin angeordneten Luftöffnung (18), die zur Verwendung in einer Dampfzirkulationsvorrichtung nach einem vorhergehenden Anspruch ausgebildet ist und enthält:-
einen abgedichteten Behälter mit einem Flüssigkeitsreservoir (65);
ein sich von dem Flüssigkeitsreservoir (65) erstreckendes Dosierventilgehäuse (74);
einen Dosierzapfen (72) mit einem Dosierelement (72a) an einem seiner Enden zum Aufnehmen und Abziehen eines festen Flüssigkeitsvolumens von dem abgedichteten Behälter (65) zum Verdampfen, wobei der Dosierzapfen (72) in dem Dosieiventilgehäuse (74) derart angeordnet ist, daß sich das Dosierelement (72a) normalerweise über das Dosierventilgehäuse (74) hinaus erstreckt und die Betätigungsmittel (50) periodisch den Dosierzapfen (72) in dem Ventilgehäuse (74) derart hin- und hergehen lassen, daß das Dosierelement (72a) in das Dosierventilgehäuse (74) eingeschoben und aus ihm mit dem festen Flüssigkeitsvolumen zurückgezogen wird;
Mittel (73) zum Wiederabdichten des Behälters (65) nach dem Zurückziehen des Dosierelementes (72a).

7. Wegwerfkartusche nach Anspruch 6, bei der der abgedichtete Behälter eine zwischen dem Reservoir und dem Dosierventilgehäuse angeordnete durchstoßbare Dichtung (68) zur Schaffung eines abgedichteten Reservoirs (65) aufweist und der Dosierzapfen (72) weiterhin ein Durchstoßelement (72c) enthält, das die durchstoßbare Dichtung (68) bei dem erstmaligen Einschieben des Dosierelements (72a) in das Dosierventilgehäuse (74) punktiert und dadurch ermöglicht, daß Flüssigkeit aus dem Flüssigkeitsreservoir unter dem Einfluß der Schwerkraft in das Dosierventilgehäuse (74) fließt.

8. Wegwerfkartusche nach Anspruch 6 oder 7, weiterhin enthaltend Mittel (74) zum Ausrichten der Kartusche, wenn sie an dem Hauptgehäuse (12) der Dampfzirkulationsvorrichtung angebracht wird.

**Revendications**

1. Appareil de circulation de vapeur, comprenant:
un boîtier principal (12), comportant une première ouverture pour de l'air (19) et un boîtier d'ensemble de recharge (14), comportant une seconde ouverture pour de l'air (18), ledit boîtier d'ensemble de recharge (14) étant relié audit boîtier principal (12);
un récipient fermé hermétiquement, comprenant un réservoir à liquide (65), monté dans le boîtier d'ensemble de recharge (14);
un boîtier de soupape de dosage (74), qui fait saillie sur le réservoir à liquide (65);
une tige de dosage (72) comprenant un élément de dosage (72a) à l'une de ses extrémités, pour collecter un volume déterminé du liquide à évaporer, la tige de dosage (72) étant située à l'intérieur dudit boîtier de soupape de dosage (74), de sorte que l'élément de dosage (72a) dépasse normalement le boîtier de soupape de dosage (74);
un moyen d'actionnement (50), pour insérer périodiquement l'élément de dosage (72a) dans le boîtier de soupape de dosage (74) et en retirer l'élément de dosage (72a), avec ledit volume déterminé de liquide;
un moyen (78) pour refermer hermétiquement le récipient (65), après que l'élément de dosage (72a) ait été retiré; et
un moyen (49) situé dans le boîtier principal (12), pour induire périodiquement un écoulement d'air, à travers lesdites ouvertures pour de l'air (18, 19), après que le récipient ait été refermé hermétiquement.

2. Appareil de circulation de vapeur selon la revendication 1, dans lequel le moyen servant à induire périodiquement un écoulement d'air est un ventilateur (49) actionné périodiquement.

3. Appareil de circulation de vapeur selon la revendication 1 ou 2, dans lequel le récipient fermé hermétiquement comprend un joint d'étanchéité transperçable (68), situé entre le réservoir (67) et le boîtier de soupape de dosage (74), pour fournir un réservoir fermé hermétiquement, ladite tige de dosage (72) comprenant un organe de perçage (72c), ledit organe de perçage (72c) perforant ledit joint d'étanchéité transperçable (68), lors de l'insertion initiale dudit élément de dosage (72a) dans ledit boîtier de soupape de dosage (74), de manière à permettre au fluide de s'écouler dudit réservoir à liquide (67), dans le boîtier de soupape de dosage (74).

4. Appareil de circulation de vapeur selon l'une quelconque des revendications précédentes, dans lequel le récipient à liquide (65) et la tige de dosage (72) sont enveloppés dans une cartouche jetable de recharge.

5. Appareil de circulation de vapeur selon la revendication 4, dans lequel ledit boîtier d'ensemble de recharge (14) enferme et fait partie de ladite cartouche jetable de recharge.

6. Cartouche jetable contenue dans un boîtier d'ensemble de recharge (14), comportant une première ouverture pour de l'air (18), ladite cartouche étant adaptée pour l'utilisation dans un appareil de circulation de vapeur selon l'une quelconque des revendications précédentes, ladite cartouche jetable comprenant:

un récipient fermé hermétiquement, comprenant un réservoir à liquide (65);

un boîtier de soupape de dosage (74), faisant saillie sur le réservoir à liquide (65);

une tige de dosage (72) comprenant, à l'une de ses extrémités, un élément de dosage (72a), pour collecter et prélever un volume déterminé dudit récipient fermé hermétiquement (65), destiné à l'évaporation, la tige de dosage (72) étant logée dans ledit boîtier de soupape de dosage (74), de sorte que l'élément de dosage (72a) dépasse normalement le boîtier de soupape de dosage (74), le moyen d'actionnement (50) déplaçant périodiquement en va-et-vient ladite tige de dosage (72), à l'intérieur dudit boîtier de soupape (74), de sorte que ledit élément de dosage (72a) est inséré dans ledit boîtier de soupape de dosage (74) et retiré de ce dernier, avec ledit volume déterminé de liquide;

un moyen (73) pour refermer hermétiquement le récipient (65), après que l'élément de dosage (72a) ait été retiré.

7. Cartouche jetable selon la revendication 6, dans laquelle ledit récipient fermé hermétiquement comprend un joint d'étanchéité transperçable (68), situé entre le réservoir et le boîtier de soupape de dosage, pour fournir un réservoir fermé hermétiquement (65), ladite tige de dosage (72) comprenant en outre un organe de perçage (72c), ledit organe de perçage (72c) perforant ledit joint d'étanchéité transperçable (68) lors de l'insertion initiale dudit élément de dosage (72a) dans ledit boîtier de soupape de dosage (74), ce qui permet ainsi au liquide de s'écouler, par gravité, dudit réservoir à liquide, dans ledit boîtier de soupape de dosage (74).

8. Cartouche jetable selon la revendication 6 ou 7, comprenant en outre un moyen (34) pour aligner la cartouche lorsqu'elle est fixée au boîtier principal (12) de l'appareil de circulation de vapeur.

FIG. 1

FIG. 2

EP 0 314 761 B1

FIG. 4

FIG. 3

10

FIG. 5